Europäisches Patentamt

European Patent Office

Office européen des brevets

(19)

(11) Numéro de publication: **0 397 571**
**A1**

## DEMANDE DE BREVET EUROPEEN

(12)

(21) Numéro de dépôt: **90401242.4**

(22) Date de dépôt: **10.05.90**

(51) Int. Cl.5: **A61K 37/16, A61K 35/20**

(30) Priorité: **11.05.89 FR 8906207**

(43) Date de publication de la demande:
**14.11.90 Bulletin 90/46**

(84) Etats contractants désignés:
**AT BE CH DE DK ES FR GB GR IT LI LU NL SE**

(71) Demandeur: **CENTRE NATIONAL DE LA RECHERCHE SCIENTIFIQUE**
**15, quai Anatole France**
**F-75007 Paris(FR)**

Demandeur: **INSTITUT DES VAISSEAUX ET DU SANG**
**Hôpital Lariboisière 8, rue Guy Patin**
**F-75010 Paris(FR)**

(72) Inventeur: **Drouet, Ludovic**
**14, avenue des Cottages**
**F-92340 Bourg la Reine(FR)**

Inventeur: **Bal Dit Sollier, Claire**
**11, Avenue Franklin Roosevelt**
**F-94300 Vincennes(FR)**
Inventeur: **Mazoyer, Elisabeth**
**85 rue du Cardinal Lemoine**
**F-75005 Paris(FR)**
Inventeur: **Lévy-Toledano, Sylviane**
**16 rue des Sablons**
**F-75116 Paris(FR)**
Inventeur: **Jollès, Pierre**
**2, rue J.F. Gerbillon**
**F-75006 Paris(FR)**
Inventeur: **Fiat, Anne-Marie**
**67 bis, avenue des Pages**
**F-78110 Le Vesinet(FR)**

(74) Mandataire: **Ayache, Monique**
**CABINET AYACHE 3, côte de la Jonchère**
**B.P. 24**
**F-92502 Rueil-Malmaison Cedex(FR)**

(54) **Utilisation du caséinoglycopeptide k pour la fabrication d'une composition, notamment d'un médicament, pour la prévention et le traitement des thromboses.**

(57) L'invention a pour objet l'utilisation du caséinoglycopeptide $x$ ou CGP, notamment de lait de vache, de brebis, de chèvre ou humain, pour la fabrication d'une composition, notamment d'un médicament, pour la prévention et/ou le traitement des thromboses.

Un médicament de ce type peut avantageusement se présenter sous forme lyophilisée destinée à l'injection, après dissolution dans un solvant compatible, ou sous forme de nanoparticules enfermées dans des gélules.

Application à la prévention et au traitement des thromboses.

## Utilisation du caséinoglycopeptide x pour la fabrication d'une composition, notamment d'un médicament, pour la prévention et le traitement des thromboses.

La présente invention a été faite au Laboratoire des Protéines de l'Université de Paris V, Laboratoire associé au Centre National de la Recherche Scientifique N° UA 1188 en collaboration avec l'Institut des Vaisseaux et du Sang.

L'invention concerne la prévention et le traitement des thromboses.

Elle a plus particulièrement pour objet l'utilisation du caséinoglycopeptide x, notamment de lait de vache, de brebis, de chèvre ou de femme, pour la fabrication d'une composition, notamment d'un médicament, pour la prévention et le traitement des thromboses.

Lors de la phase primaire de la coagulation du lait, la chymosine (EC 3.4.23.4) scinde spécifiquement une liaison entre deux acides aminés à l'intérieur de la caséine x de vache, à savoir la liaison entre la phénylalanine et la méthionine correspondant aux résidus 105→106. Il y a alors libération du caséinoglyco-peptide x ou CGP (partie C-terminale de la caséine x ; résidus 106→169), également appelé caséinomacro-peptide.

Ce caséinoglycopeptide x qui est donc un peptide naturel, s'obtient également par d'autres traitements de la caséine entière, par exemple par traitement chimique ou par la chaleur. Lors de la digestion du lait, le caséinoglycopeptide x est le premier peptide libéré à l'intérieur du complexe appelé caséine entière ($\alpha_{S1}$, $\alpha_{S2}, \beta, x$).

Dans l'article référencé Eur. J. Biochem. 158, 379-382 (1986), Pierre JOLLES, Sylviane LEVY-TOLEDANO, Anne-Marie FIAT, Claudine SORIA, Dieter GILLESSEN, Annick THOMAIDIS, Fred W.DUNN et Jacques P. CAEN ont étudié, entre autres, l'aptitude de trois fragments du caséinoglycopeptide x de lait de vache, à savoir les fragments 106→112, 113→116 et 106→116 obtenus soit par digestion du CGP par la trypsine (EC 3.4.21.4) soit par synthèse, en ce qui concerne leur aptitude à inhiber in vitro l'agrégation plaquettaire et ou la liaison du fibrinogène aux plaquettes.

Les résultats qu'ils ont ainsi obtenus sont très variables d'un peptide à l'autre. Ainsi ont-ils pu observer qu'in vitro le undécapeptide 106→116 du caséinoglycopeptide x de lait de vache inhibe tant la liaison du fibrinogène aux plaquettes que l'agrégation de ces dernières. En revanche, le heptapeptide contenant les résidus 106→112 peut être considéré comme n'inhibant pas l'agrégation plaquettaire, tandis que le tétrapeptide contenant les résidus 113→116 n'est que très faiblement inhibiteur de l'agrégation plaquettaire. Au surplus, ni l'un ni l'autre de ces peptides n'inhibe la liaison du fibrinogène aux plaquettes, du moins aux mêmes concentrations que le undécapeptide 106→116 qui, rappelons le, est constitué de la combinaison de ce hepta- et de ce tétrapeptide.

Ces résultats apparemment contradictoires, ou pour le moins disparates, qui plus est obtenus seulement in vitro sur des fragments peptidiques du CGP, ne pouvaient aucunement laisser prévoir que le caséinoglycopeptide x, notamment de lait de vache, de brebis, de chèvre ou de femme pourrait présenter une activité antithrombotique in vivo , et ceci d'autant moins qu'il est bien connu de l'homme du métier que l'activité d'un composé sur la fonction plaquettaire, en particulier in vitro , n'implique pas obligatoirement une activité antithrombotique in vivo .

Ceci est confirmé notamment par le fait que la SOCIETE DES PRODUITS NESTLE S.A. qui cherchait à valoriser les caséinoglycopeptides x d'origine bovine, caprine ou ovine n'a, dans sa demande de brevet EP 0 283 675 déposée le 29 janvier 1988 et revendiquant la priorité d'un dépôt antérieur en Suisse du 26 février 1987, c'est-à-dire postérieur à la date de publication de l'article de Pierre JOLLES et coll. mentionné ci-dessus, fait aucune référence à une éventuelle activité antithrombotique de ces composés. Les activités anti-plaque et anti-carie dentaires décrites in vitro dans cette demande de brevet, et dont l'application est revendiquée, n'ont rien à voir avec une activité antithrombotique in vivo.

De même, la demande de brevet EP 0 291 264 de la société SNOW BRAND MILK PRODUCTS CO. LTD, déposée le 9 mai 1988 et revendiquant la priorité d'une demande de brevet japonaise du 15 mai 1987, qui concerne un procédé industriel de production de glycomacropeptide de caséine x, ne fait aucune référence à une éventuelle application de ce glycopeptide à la prévention ou au traitement des thromboses. Elle fait seulement référence à l'utilisation connue du glycomacropeptide de la caséine x en tant qu'agent protecteur contre l'infection et revendique l'utilisation du glycomacropeptide de la caséine x , obtenu par le procédé qu'elle décrit, pour la préparation d'un agent anorexiant utilisable dans des "programmes" de contrôle du poids.

Les demandeurs ont maintenant établi que le caséinoglycopeptide de caséine x, appelé ci-après CGP, notamment de lait de vache, de brebis, de chèvre ou de femme, agit non seulement sur certaines fonctions plaquettaires in vitro mais encore présente une activité antithrombotique in vivo.

Plus précisément, l'activité sur les fonctions plaquettaires a été étudiée *in vitro* en ce qui concerne l'inhibition de l'agrégation des plaquettes provoquée par l'ADP ("agrégation à l'ADP"), l'inhibition de la liaison du fibrinogène aux plaquettes provoquée par l'ADP ("liaison du fibrinogène à l'ADP"), l'inhibition de l'agrégation des plaquettes provoquée par la thrombine ("agrégation à la thrombine") et l'inhibition de la réaction provoquée par la thrombine ("réaction de sécrétion à la thrombine") c'est-à-dire la libération d'un des constituants du granule dense (sérotonine), témoin de l'activation plaquettaire.

Cette activité du CGP sur les fonctions plaquettaires a été comparée à celle de la caséine *x* et de la paracaséine *x* . Il est rappelé à ce propos que la caséine *x* fournit, sous l'action notamment de la chymosine, la paracaséine *x* (fragment 1→105) et le caséinoglycopeptide K ou CGP (fragment 106→169). Les essais effectués sont décrits plus en détail dans la partie expérimentale du présent mémoire.

Dans tous les essais effectués, la paracaséine *x* est inactive. La caséine *x* inhibe seulement l'agrégation à la thrombine et la réaction de sécrétion à la thrombine (inhibition de 50 % à une concentration 10 $\mu$M dans chaque cas). Le CGP provoque les mêmes inhibitions, aux mêmes concentrations mais est également capable de provoquer, à une concentration 250 $\mu$M, une inhibition de 50 % de l'agrégation à l'ADP, alors que la caséine *x* est inactive dans cet essai.

Par ailleurs ,l'activité antithrombotique *in vivo* a été démontrée chez le rat et le cobaye selon un protocole classique, explicité dans la partie expérimentale qui suit.

L'invention a donc pour objet l'utilisation du caséinoglycopeptide *x*, notamment de lait de vache, de brebis, de chèvre ou de femme, pour la fabrication d'une composition, notamment d'un médicament, pour la prévention et le traitement des thromboses.

Comme il a été indiqué plus haut, le caséinoglycopeptide *x*, notamment de lait de vache, de brebis, de chèvre ou de femme, peut être obtenu par différents procédés connus de la littérature, et en particulier selon la technique décrite dans la partie expérimentale du présent mémoire et qui peut se résumer comme suit.

Du lait délipidé est soumis à une dialyse contre de l'eau pour en retirer les sels et les sucres libres. Le pH du lait ainsi traité est amené au point isoélectrique (4,6) au moyen d'un acide tel que HCl M, le lait est ensuite avantageusement chauffé modérément (à environ 40° C) pour augmenter la précipitation de la caséine puis centrifugé, par exemple à 3000 tours/minute, pour séparer la caséine.

La caséine, notamment après lyophilisation, est mise en solution à pH 6,8 , par exemple dans du tampon pyridine 0,2N/acide acétique. Elle est ensuite digérée par un enzyme tel que la chymosine. Lorsque l'action de l'enzyme est terminée, le mélange est traité par un acide tel que l'acide trichloroacétique, ce qui provoque la formation d'un précipité. Le surnageant qui contient le CGP est séparé par centrifugation. Le surnageant de centrifugation est à son tour filtré et lavé à l'éther. La phase aqueuse purifiée contient le CGP qui peut être conservé à l'état lyophilisé. Dans certains cas, notamment dans le cas du caséinoglyco-peptide *x* de lait humain, une purification supplémentaire, notamment par chromatographie sur colonne échangeuse d'anions, peut être avantageuse, voire nécessaire.

Un processus analogue peut s'appliquer à la séparation de tout autre caséinoglycopeptide *x* de lait de mammifère.

La composition, notamment le médicament, faisant l'objet de l'invention, peut être obtenue en incorporant le CGP dans une forme, notamment pharmaceutique, convenable en présense d'excipients et/ou diluants inertes et éventuellement d'autres principes actifs.

En tant que formes pharmaceutiques, on peut citer :
- les formes pharmaceutiques convenant à l'administration par voie intraveineuse, telles que la forme lyophilisée reconstituée dans un solvant compatible tel qu'une solution aqueuse stérile et apyrogène de chlorure de sodium à 9 ‰, et
- les formes pharmaceutiques convenant à l'administration par voie orale telles que des gélules, notamment gastrorésistantes, des nanosphères , des nanoparticules et des liposomes.

Les nanosphères contenant le CGP sont avantageusement constituées d'une ou plusieurs matières permettant au CGP de passer la barrière digestive, telles que par exemple l'isobutylcyanoacrylate.

Les gélules qui peuvent contenir de telles nanosphères peuvent avantageusement être constituées d'une matière gastrorésistante telle que l'acétophtalate de cellulose ou une résine méthacrylique.

D'autres formes pharmaceutiques et d'autres voies d'administration peuvent être envisagées, sur la base des connaissances usuelles de l'homme du métier en la matière.

Les médicaments selon l'invention peuvent être utilisés dans la prophylaxie et/ou le traitement des thromboses.

Dans le cas de la prophylaxie des thromboses, la posologie usuelle chez l'adulte est avantageusement de 15 à 30 mg/kg de poids corporel, 2 fois par jour par voie intraveineuse, et de 50 à 100 mg/kg de poids corporel, 2 fois par jour par voie orale.

Compte tenu de l'absence de toxicité du CGP, ces posologies peuvent être dépassées si besoin est. Elles peuvent aussi être diminuées, suivant la sensibilité et/ou la tolérance du patient.

Dans le cas du traitement des thromboses, la posologie usuelle chez l'adulte est avantageusement de 30 à 50 mg/kg de poids corporel, par jour, par voie intraveineuse continue, et de 50 à 100 mg/kg de poids corporel, 2 fois par jour, par voie orale.

Ici encore, ces posologies moyennes peuvent être augmentées ou diminuées selon l'état à traiter, par exemple.

Les exemples non limitatifs dont la description suit sont destinés à illustrer l'invention.


## PARTIE EXPERIMENTALE


### I Préparation :


#### 1) Préparation des caséines de vache, de brebis, de chèvre ou de femme.

Du lait de vache, de brebis, de chèvre ou de femme est congelé à -20° C. Lors de la préparation de la caséine, le lait est décongelé et quelques gouttes de toluène sont ajoutées pour éviter la prolifération des bactéries. Pour retirer les graisses, le lait est centrifugé pendant une heure à 3000 tours/min. Le lait est ensuite refroidi à 4° C et maintenu 3 heures à 4° C. Une filtration sur coton permet de séparer les graisses du lait délipidé. Une dialyse contre de l'eau permet de retirer les sels et les sucres libres du lait. Le pH du lait est abaissé jusqu'au point isoélectrique de la caséine, à savoir 4,6 , au moyen de HCl M. Pour augmenter la quantité du précipité, la température est élevée à 40° C pendant une demi-heure. Une centrifugation à 3000 tours/min pendant une demi-heure sépare la caséine des autres constituants du lait. La caséine est ensuite lavée avec de l'eau distillée puis recentrifugée, toujours à 3000 tours/min pendant une demi-heure. Le précipité qui correspond à la caséine est lyophilisé.


#### 2) Préparation des caséinoglycopeptides $x$ de vache, de brebis, de chèvre ou de femme.

La caséine (2 %) est mise en solution dans un tampon pyridine 0,2N/acide acétique M, pH 6,8. La digestion enzymatique de la caséine est réalisée grâce à la chymosine (E/S = 1/2000) (Sigma) pendant une heure trente à 37° C. On ajoute de l'acide trichloroacétique jusqu'à une concentration finale de 12 %. Un précipité se forme après 18 heures à 4° C. Le caséinoglycopeptide $x$ se trouve dans le surnageant et est séparé du précipité par centrifugation pendant 2 heures à 3000 tours/min. Le surnageant est recueilli après passage sur verre fritté. Pour éliminer l'acide trichloroacétique dans le surnageant, on effectue des lavages à l'éther. Le surnageant qui contient le caséinoglycopeptide $x$ est ensuite lyophilisé.

Pour le caséinoglycopeptide $x$ de femme, une étape supplémentaire pour la purification est nécessaire. Elle consiste en une chromatographie sur une colonne échangeuse d'anions (HR 16/10 Mono Q, Pharmacia), à l'aide d'un appareil FPLC (Fast Performance Liquid Chromatography) de la société Pharmacia. L'éluant A, dans l'essai effectué, était constitué d'une solution tampon Tris-HCl 0,04M, pH 7,6 puis un gradient contenant la solution A et du NaCl M a été ajouté progressivement. La fraction correspondant au caséinoglycopeptide $x$ a été désalifiée sur une colonne de filtration de Sephadex ® G-25 (1900 x 25 mm) (Pharmacia) avec de l'acide acétique à 30% comme éluant. Le caséinoglycopeptide $x$ a ensuite été lyophilisé.


#### 3) Analyse des caséinoglycopeptides $x$ de vache, de chèvre, de brebis et humain.

Les séquences de ces différents caséinoglycopeptides $x$ ont été décrites dans la littérature scientifique, à savoir :
- caséinoglycopeptide $x$ de vache : J. Jollès et coll., FEBS letters 30 (1973) 173-175;
- caséinoglycopeptide $x$ de brebis : J. Jollès et coll., Biochim. Biophys. Acta 365 (1974) 335-343;
- caséinoglycopeptide $x$ de chèvre : J.C. Mercier et coll., Biochimie, 58 (1976) 1303-1310;
- caséinoglycopeptide $x$ humain : J.M. Chobert et coll., FEBS Letters, 72 (1976) 173-178.

Après hydrolyse totale par HCl 6N + mercapto-2-éthanol au 1/2000 pendant 18 heures à 110° C sous vide, on a vérifié que les compositions en acides aminés (résidus/mole) étaient bien celles attendues (voir le tableau I qui suit).

TABLEAU I

| Composition en acides aminés des caséinoglycopeptides $x$ (résidus /mole) | | | | |
|---|---|---|---|---|
| | Vache | Brebis | Humain | Chèvre |
| Asp | 5 | 7 | 3 | 7 |
| Thr | 12 | 10 | 14 | 11 |
| Ser | 6 | 7 | 4 | 8 |
| Glu | 10 | 9 | 6 | 9 |
| Pro | 8 | 7 | 11 | 6 |
| Gly | 1 | 0 | 0 | 0 |
| Ala | 5 | 10 | 7 | 9 |
| Val | 6 | 6 | 6 | 5 |
| Met | 1 | 1 | 0 | 1 |
| Ile | 6 | 5 | 10 | 6 |
| Leu | 1 | 0 | 0 | 0 |
| Phe | 0 | 0 | 1 | 0 |
| Lys | 3 | 3 | 3 | 3 |
| His | 0 | 1 | 0 | 1 |

La séquence N -terminale de ces quatre caséinoglycopeptides $x$ a été déterminée et a permis de vérifier la pureté de ces produits.

## Caséinoglycopeptide

**Vache:** Met - Ala - Ile - Pro - Pro - Lys - Lys - Asn - Gln - Asp - Lys

**Brebis:** Met - Ala - Ile - Pro - Pro - Lys - Lys - Asp - Gln - Asp - Lys

**Chèvre:** Met - Ala - Ile - Pro - Pro - Lys - Lys - Asp - Gln - Asp - Lys

**Femme:** Ile - Ala - Ile - Pro - Pro - Lys - Lys - Ile - Gln - Asp - Lys

II - Etude biochimique

1) Durée de vie du caséinoglycopeptide $x$ de vache dans le plasma à 37° C

100 μg de caséinoglycopeptide x de vache ont été introduits dans 100 μl de plasma de rat et incubés à 37°C pendant différents temps (0, 1 min, 5 min, 15 min, 30 min, 45 min, 1 h, 1 h15 et 1 h30). Il a été ajouté dans chaque tube, à la fin de l'incubation, de l'acide perchlorique à la concentration finale de 6%. Un précipité s'est formé et une centrifugation de 15 min à 5000 tr/min a permis de séparer le surnageant du précipité. 20 μl de surnageant ont été chromatographiés par HPLC sur un appareil Waters avec une colonne de phase inverse RP 300 (4,6 x 220 mm, Brownlee Labs). Le débit était de 1 ml. Les conditions d'élution ont été les suivantes : pendant 10 min du solvant A (acide trifluoroacétique à 0,1% dans l'eau) a été utilisé seul puis un gradient linéaire constitué de solvant A et de solvant B (acide trifluoroacétique à 0,1% dans l'acétonitrile) a été ajouté progressivement pendant 60 min.

Le caséinoglycopeptide x de vache a été élué à 40% de solvant B et détecté à 220 nm. Le plasma seul traité dans les mêmes conditions a montré qu'aucun pic n'était détecté au même niveau que celui du caséinoglycopeptide x. Il a été trouvé que la surface du pic du caséinoglycopeptide x était identique aux temps 1 min, 5 min, 15 min, 30 min, 45 min et 1h. Il a été observé ensuite une diminution du pic de l'ordre de 15% à 1h 15 et de 30 % à 1h 30.

On en conclut que le caséinoglycopeptide x de vache est stable dans le plasma pendant 1h et qu'ensuite il y a une dégradation lente.

2) Identification du caséinoglycopeptide x de vache dans le plasma après son injection *in bolus, in vivo,* chez le rat

Il a été injecté dans la verge du rat *in bolus* du caséinoglycopeptide de vache à raison de 5 mg/kg d'animal. Des prélèvements de sang ont été effectués à différents temps et ont été centrifugés à 2000 tr/min pendant 15 min afin d'obtenir le plasma. On a recherché dans le plasma, par HPLC, dans les mêmes conditions que celles décrites sous II 1), le pic correspondant au caséinoglycopeptide x qui a été retrouvé à 1 min, 5 min, 15 min, 30 min, 45 min, 1h, 1h 15 et 1h30. Cependant la surface du pic était deux fois plus importante à 1 min qu'à 5 min.

On a observé ensuite une légère diminution de ce pic au cours du temps (entre 1 min et 90 min).

Pour identifier réellement le caséinoglycopeptide x dans le plasma, le pic d'HPLC correspondant à l'emplacement du caséinoglycopeptide x prélevé sur les plasmas recueillis à 1 min et à 90 min après le *bolus* a été séquencé et, dans les deux cas, on a retrouvé la séquence des 10 premiers acides aminés du caséinoglycopeptide x de vache, soit :

Met - Ala - Ile - Pro - Pro - Lys - Lys - Asn - Gln - Asp

Cependant la quantité du caséinoglycopeptide x de vache était plus faible pour le plasma recueilli 90 min après le *bolus* que pour celui à 1 min.

On en conclut que le caséinoglycopeptide x de vache, injecté *in bolus* chez le rat, est retrouvé intact, sans hydrolyse, dans le plasma pendant au moins 90 min.

III - Etude pharmacologique

1) Etude pharmacologique comparative *in vitro* de l'activité du CGP sur les fonctions plaquettaires.

L'action de la chymosine sur la caséine x de vache donne naissance à deux peptides : la paracaséine x (1→105) et le caséinoglycopeptide x ou CGP(106→169).

Ces trois produits ( caséine x, paracaséine x et CGP) ont été testés sur l'agrégation des plaquettes humaines à l'ADP et à la thrombine, sur la fixation du fibrinogène en présence d'ADP et sur la réaction de sécrétion.

Le tableau II qui suit résume les résultats obtenus avec ces trois produits. La caséine x n'inhibe ni l'agrégation à l'ADP, ni la liaison du fibrino gène. Elle inhibe l'agrégation à la thrombine ainsi que la réaction de sécrétion avec une CI$_{50}$ (concentration inhibant 50 % de l'agrégation par rapport au témoin) 10 μM environ. La paracaséine x n'a aucun effet sur aucune des fonctions plaquettaires testées.

Le caséinoglycopeptide x inhibe l'agrégation à l'ADP. La concentration inhibitrice à 50 % (CI$_{50}$) se

situe autour de 250 $\mu$M. A cette concentration, aucune inhibition de la liaison du fibrinogène n'est obtenue.

Ce caséinoglycopeptide est également un inhibiteur très puissant de l'agrégation à la thrombine ainsi que de la réaction de sécrétion, puisque la concentration inhibitrice à 50 % se situe autour de 10 $\mu$M.

TABLEAU II

| Effets comparés de la caséine $x$, de la paracaséine $x$ et du CGP sur différentes fonctions plaquettaires. | | | | |
|---|---|---|---|---|
| | Agrégation à l'ADP | Liaison du fibrinogène à l'ADP | Agrégation à la thrombine | Réaction de sécrétion à la thrombine |
| Caséine $x$ | -- | -- | 10 $\mu$M | 10 $\mu$M |
| Paracaséine $x$ | -- | -- | -- | -- |
| Caséinoglycopeptide ou CGP | 250 $\mu$M | -- | 10 $\mu$M | 10 $\mu$M |
| Notes : Les valeurs indiquées correspondent aux concentrations des produits qui induisent 50 % d'inhibition des différentes fonctions.<br>-- : pas d'inhibition. | | | | |

2). Etude pharmacologiqe *in vivo* : effet du CGP sur la thrombogénèse chez le rat.

L'action du caséinoglycopeptide $x$ (CGP) de vache a été étudiée *in vivo*, chez le rat.

A. Méthode

a. Thrombose expérimentale

*In vivo* , dans le modèle utilisé, la thrombogénèse est induite par une lésion de l'endothélium des artérioles du mésentère de l'animal, par un rayon laser colorant, pompé par un laser azote pulsé. Le phénomène de thrombose est un phénomène cyclique répétitif. Plusieurs vaisseaux sont étudiés après une injection i.v. du peptide à tester. Chaque vaisseau est observé 15 minutes durant lesquelles on compte le nombre de thromboses formées. L'expérience dure au total environ 1h30 après l'injection i.v. du peptide.

b. Animaux

Les animaux utilisés sont des rats mâles de race Sprague-Dawley, d'environ 500g.

B. Résultats : Thrombose expérimentale induite

Ils peuvent etre estimés grâce à deux paramètres :
- le pourcentage d'inhibition du phénomène de thrombogénèse; celui-ci étant en général à son maximum pendant les 20 min suivant l'injection i.v. ;
- le temps d'action du peptide durant lequel on observe encore un pourcentage d'inhibition de la thrombogénèse significatif par rapport au témoin.

Chez le rat, à 0,5 $\mu$M/kg (5000 $\mu$g/kg) on observe un effet antithrombotique significatif: 1,4 thromboses/15 min pendant les 20 premières minutes d'observation (témoin : 4 thromboses/15min), soit 65% d'inhibition du phénomène de thrombogénèse. Cet effet reste significatif jusqu'à 1h30 après l'injection i. v. du CGP.

3) Etude pharmacologique *in vivo* : effet du CGP sur la thrombogénese chez le cobaye.

L'étude *in vivo* du CGP du lait de vache sur le rat a été approfondie par une étude similaire sur le cobaye. On a étudié non seulement l'effet du CGP du lait de vache mais aussi des différents CGP issus du lait de brebis, de chèvre et humain.

Les CGP ont été testés à une ou deux doses :
- 0,5 mg/kg soit 0,05 µM/kg
- 1 mg/kg soit 0,1 µM/kg

## A. Méthode

### a) Thrombose expérimentale

On a procédé de façon identique à ce qui a été fait chez le rat, à savoir : lésions de l'endothélium et thromboses induites par un laser colorant, pompé par un laser azote pulsé.

### b) Animaux

Les animaux utilisés sont des cobayes mâles de race Dunking-Hartley d'environ 400 g.

## B. Résultats

Les résultats obtenus sont résumés dans le tableau III qui suit.

TABLEAU III

| PEPTIDE | CGP BREBIS | CGP HUMAIN | CGP CHEVRE | CGP VACHE |
|---|---|---|---|---|
| dose (mg/kg) | 0,5 1 | 0,5 1 | 0,5 1 | 0,5 1 |
| inhibition maximum (%) | 45 70 | 40 90 | NT 45 | NT 50 |
| durée d'action (min) | 100 100 | 60 100 | NT 40 | NT 100 |
| NT : non testé | | | | |

## IV Exemples de préparations pharmaceutiques :

### A- Exemple de préparation pour injection intraveineuse :

On prépare deux flacons à mélanger extemporanément :
- un flacon A contenant de 100 à 300 mg d'une préparation stérile et apyrogène de caséinoglycopeptide *x* lyophilisée, et
- un flacon B contenant une solution stérile et apyrogène de chlorure de sodium à 9 pour mille.

Au moment de l'emploi, le flacon B est repris stérilement dans une seringue puis introduit dans le flacon A; la dilution du caséinoglycopeptide *x* y est instantanée. Le caséinoglycopeptide *x* en solution est repris dans la seringue et, par un changement de tubulure, utilisé tel quel pour l'injection au malade, selon une dose adaptée au poids du malade.

B - Exemple de préparation pour absorption *per os* :

Le caséinoglycopeptide *x* est enrobé dans des nanoparticules d'isobutylcyanoacrylate pour lui permettre de passer la barrière digestive.

Pour éviter l'acidité gastrique, la suspension de nanoparticules est lyophilisée et enrobée dans des gélules d'acétophtalate de cellulose ou dans une résine méthacrylique.

Les gélules sont dosées à 100 mg de caséinoglycopeptide *x*.

La prise est adaptée au poids du malade. Elle est répartie sur la journée en faisant attention aux prises médicamenteuses, par rapport aux prises alimentaires.

## Revendications

1 . Utilisation du caséinoglycopeptide *x* ou CGP, notamment de lait de vache, de brebis, de chèvre ou humain, pour la fabrication d'une composition, notamment d'un médicament, pour la prévention et/ou le traitement des thromboses.

2 . Utilisation du caséinoglycopeptide *x* ou CGP, notamment de lait de vache, de brebis, de chèvre ou humain, pour la fabrication d'un médicament pour la prévention et/ou le traitement des thromboses.

3 . Utilisation selon la revendication 2, pour la fabrication d'un médicament utilisable dans la prévention des thromboses, à une posologie de 15 à 30 mg/kg de poids corporel, 2 fois par jour par voie intraveineuse , ou pour le traitement des thromboses, à une posologie de 30 à 50 mg/kg de poids corporel, par jour, par voie intraveineuse continue.

4 . Utilisation selon la revendication 3, caractérisée en ce que le médicament se présente sous forme lyophilisée, pour être reconstitué dans un solvant compatible, de préférence une solution aqueuse stérile et apyrogène de chlorure de sodium à 9‰.

5 . Utilisation selon la revendication 2, pour la fabrication d'un médicament utilisable dans la prévention des thromboses, à une posologie de 50 à 100 mg/kg de poids corporel, 2 fois par jour, par voie orale, ou dans le traitement des thromboses, à une posologie de 50 à 100 mg/kg de poids corporel, 2 fois par jour, par voie orale.

6 . Utilisation selon la revendication 5, caractérisée en ce que le médicament se présente sous forme de gélules, de préférence gastrorésistantes, de nanosphères ou de liposomes.

7 . Utilisation selon la revendication 6, caractérisée en ce que les nanosphères contenant le caséinoglycopeptide *x* ou CGP sont constituées d'une ou plusieurs matières gastrorésistantes permettant au CGP de passer la barrière digestive et comprenant de préférence de l'isobutylcyanoacrylate.

8 . Utilisation selon la revendication 6, dans laquelle les gélules qui contiennent de préférence des nanosphères selon la revendication 7, sont constituées d'une matière gastrorésistante, de préférence d'acétophtalate de cellulose ou d'une résine méthacrylique.

9 . Utilisation selon la revendication 3 ou 4, caractérisée en ce que l'on prépare deux flacons à mélanger extemporanément :
- un flacon contenant 100 à 300mg d'une préparation stérile et apyrogène de caséinoglycopeptide *x* ou CGP, lyophilisée; et
- un flacon contenant une solution stérile et apyrogène de chlorure de sodium à 9 pour mille.

10 . Utilisation selon l'une des revendications 5 à 8, caractérisée en ce que l'on enrobe le caséinoglycopeptide *x* ou CGP dans des nanoparticules d'isobutylcyanoacrylate, on lyophilise la suspension de nanoparticules et on l'introduit dans des gélules d'acétophtalate de cellulose ou dans une résine méthacrylique, chaque gélule contenant 100 mg de CGP.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X,D | European Journal of Biochemistry vol. 158, 1986, pages 379 - 382; JOLLES Pierre et al: "Analogy between fibrinogen and casein" * le document en entier * | 1-2 | A61K37/16 A61K35/20 |
| A | * le document en entier * --- | 3-10 | |
| A | Biological Abstract Nb 67052203 KAYE N et al: " Characterization of the amino-acid of bovin fibrinogen ...." & MOL.CELL.BIOCHEM.,1978, (20),no3,173-82 --- | 1-10 | |
| A,D | EP-A-291264 (SNOW BRAND MILK PRODUCTS) * le document en entier * --- | 1-10 | |
| A,D | EP-A-283675 (NESTLE) * le document en entier * ----- | 1-10 | |

| | |
|---|---|
| | DOMAINES TECHNIQUES RECHERCHES (Int. Cl.5 ) |
| | A61K |

Le présent rapport a été établi pour toutes les revendications

| Lieu de la recherche | Date d'achèvement de la recherche | Examinateur |
|---|---|---|
| BERLIN | 20 JUIN 1990 | AVEDIKIAN P.F. |